# EUROPEAN PATENT APPLICATION

(11) **EP 3 476 277 A1**
(43) Date of publication of application: **01.05.2019**
(21) Application number: 18177792.1
(22) Date of filing: 14.06.2018
(51) Int. Cl.: A61B 5/00, A61B 10/00

(54) **SENSOR ASSEMBLY FOR SELF-DIAGNOSIS OF BREAST CANCER**

(30) Priority: 30.10.2017 KR 20170142838
(71) Applicant: Microdisplay Co., Ltd., Gyeonggi-do 14449 (KR)
(72) Inventor: KIM, Ki Young, 06285 Seoul (KR); JUNG, Sang Shin, 14449 Gyeonggi-do (KR)
(74) Representative: Bungartz Christophersen Partnerschaft mbB Patentanwälte

(57) **Abstract**

The invention relates to a sensor assembly for self-diagnosis of breast cancer, the sensor assembly includes a poly-di-methyl-siloxane (PDMS) module sensing elasticity distribution of breast tissue and emitting near-infrared rays to allow a mobile analyzing device to obtain and process tactile images and near-infrared images for self-diagnosis of breast cancer, and a mounting module to which the PDMS module is detachably mounted in a screw-coupling manner in which the mounting module wirelessly supplies an operating power to the PDMS module through contact between electrodes.

## Description

### CROSS REFERENCE TO RELATED APPLICATION(S)

This application claims the benefit of Korean Patent Application No. 10-2017-0142838, filed on October 30, 2017, which is hereby incorporated by reference in its entirety into this application.

### BACKGROUND OF THE INVENTION

### Field of the Invention

The present invention relates generally to a sensor assembly for self-diagnosis of breast cancer. Particularly, the present invention relates to a sensor assembly which is attached to a skin around user's breast to self-diagnose breast cancer, wherein the sensor assembly includes a poly-di-methyl-siloxane (PDMS) module and a mounting module to which the PDMS module is detachably mounted in a screw-coupling manner in which the mounting module wirelessly supplies an operating power to the PDMS module through contact between electrodes.

### Description of the Related Art

Generally, breast cancer is cancer that occurs mainly in women in their 40s and needs early diagnosis and treatment in order to reduce the death rate. However, in the early of the occurrence of the breast cancer, most of patients do not feel subjective symptoms, but often find a pain-free lump through a tactile self-examination. Results of such tactile self-examinations may differ according to patient's self-diagnostic proficiency and sensitivity, so a regular medical checkup is recommended.

A breast cancer-imaging apparatus includes an X-ray mammographic apparatus, an ultrasonic scanner, a magnetic resonance imaging (MRI) apparatus, etc., among which the X-ray mammographic apparatus (disclosed in KR 10-2009-0096934 and 10-2008-0004564) is widely used in a regular medical checkup for early diagnosis of breast cancer. The X-ray mammographic apparatus uses a phenomenon that X-ray transmissivity differs according to breast tissue, so as to find breast tissue absorbing X-rays, particularly, a calcified tissue, for example. The calcified tissue is likely to develop into cancer tissue, so early fining of the calcified tissue also helps preventing breast cancer.

However, since the X-ray mammographic apparatus tends to find breast cancer tissue by a visual inspection of abnormal breast tissue which is less distinguishable from normal breast tissue, diagnosis by a cancer specialist is essentially required. Thus, the X-ray mammographic apparatus has problems in that the diagnostic precision differs according to the specialist's proficiency, and inspection costs are expensive. In addition, there are other problems such as restrictive 2D monitoring using electromagnetic radiation having a wavelength of less than that of violet rays, the risk of radiation exposure, and the like. Particularly, as the incidence of breast cancer increases with westernized eating habits, it is required to overcome the problems with the conventional X-ray mammographic apparatus and to develop a digitized self-diagnostic system for breast cancer.

The foregoing is intended merely to aid in the understanding of the background of the present invention, and is not intended to mean that the present invention falls within the purview of the related art that is already known to those skilled in the art.

### SUMMARY OF THE INVENTION

Accordingly, the present invention has been made keeping in mind the above problems occurring in the related art, and an object of the present invention is to propose a sensor assembly for self-diagnosis of breast cancer, wherein the sensor assembly is attached to a skin around user's breast to self-diagnose breast cancer, wherein the sensor assembly includes a poly-di-methyl-siloxane (PDMS) module and a mounting module to which the PDMS module is detachably mounted in a screw-coupling manner in which the mounting module wirelessly supplies an operating power to the PDMS module through contact between the electrodes.

Another object of the present invention is to provide a sensor assembly for self-diagnosis of breast cancer, wherein the sensor assembly includes a poly-di-methyl-siloxane (PDMS) module and a mounting module to which the PDMS module is detachably mounted in a screw-coupling manner in which the mounting module wirelessly supplies an operating power to the PDMS module through contact between electrodes, thereby having the effects that the sensor assembly can be easily used, and when in not use, simple screw-detachment of the PDMS module from the mounting module enables the PDMS module to be turned off to save power.

In order to achieve the above objects, according to one aspect of the present invention, there is provided a sensor assembly for self-diagnosis of breast cancer, the sensor assembly including:
- a poly-di-methyl-siloxane (PDMS) module sensing elasticity distribution of breast tissue and emitting near-infrared rays to allow a mobile analyzing device to obtain and process tactile images and near-infrared images for self-diagnosis of breast cancer; and
- a mounting module to which the PDMS module is detachably mounted in a screw-coupling manner in which the mounting module wirelessly supplies an operating power to the PDMS module through contact between electrodes.

The PDMS module may include:
- a streamlined casing having a tactile sensor mounted in a rectangular recess of the casing, a plurality of first LED holders respectively provided in four inner walls of the rectangular recess, and a screw part provided on a lower side of the casing so as to be screw-coupled with the mounting module;
- an LED holder frame fixedly inserted into the casing and having a plurality of second LED holders corresponding to the first LED holders; and
- an LED-mounting frame fixedly inserted into the casing to which the LED holder frame has been mounted, and having four LED brackets to which four near-infrared LEDs are respectively mounted to emit near-infrared rays towards breast tissue so as to obtain near-infrared images of the breast tissue, a power connecting PCB plate to which the four LED brackets are mounted, and a fixing protrusion provided on a lower side of the power connecting PCB plate.

The first LED holders and the second LED holders may serve to maintain a direction of near-infrared rays emitted from the near-infrared LEDs without scattering and leaking of the near-infrared rays.

The first LED holder and the second LED holder may vertically join together to form an LED-mounting hole through which the near-infrared LED mounted to the LED bracket is fixedly held without additional components.

The four near-infrared LEDs may be fixedly mounted at 90 degree angles to the LED brackets, so that the four near-infrared LEDs are able to emit near-infrared rays towards the tactile sensor of the PDMS module at the same height and direction.

The power connecting PCB plate of the LED-mounting frame may be additionally provided with contact electrodes, which are to be connected to a power source of the mounting module when the PDMS module is screw-coupled with the mounting module through the screw part of the casing, and the contact electrodes are to be disconnected from the power source of the mounting module when the PDMS module is unscrewed from the mounting module.

The mounting module may include:
- an upper casing part having a streamlined shape, the upper casing part being detachable screw-coupled with the screw part of the PDMS module;
- a main PCB plate disposed in the upper casing part while having a shape corresponding to the upper casing part, the main PCB plate having an electrode for supplying an operating power to the PDMS module, and a fixing hole into which the fixing protrusion is positively inserted; and
- a base frame accommodating the main PCB plate when coupled to the upper casing part.

The mounting module may additionally include a clip-type device holder fastened to one side of the base frame to hold a mobile analyzing device.

According to the present invention, the sensor assembly for self-diagnosis of breast cancer is configured such that the sensor assembly is attached to a skin around user's breast to self-diagnose breast cancer, and the sensor assembly includes the PDMS module and the mounting module to which the PDMS module is detachably mounted in a screw-coupling manner in which the mounting module wirelessly supplies an operating power to the PDMS module through contact between electrodes, thereby providing simple structure and ease use of the sensor assembly.

Further, according to the present invention, the sensor assembly includes the PDMS module and the mounting module to which the PDMS module is detachably mounted in a screw-coupling manner in which the mounting module wirelessly supplies an operating power to the PDMS module through contact between electrodes, thereby having the effects that the sensor assembly can be easily used, and when in not use, simple screw-detachment of the PDMS module from the mounting module enables the PDMS module to be turned off to save power.

### BRIEF DESCRIPTION OF THE DRAWINGS

The above and other objects, features and advantages of the present invention will be more clearly understood from the following detailed description when taken in conjunction with the accompanying drawings, in which:
- FIG. 1: is an exploded perspective view illustrating a poly-di-methyl-siloxane (PDMS) module of a sensor assembly for self-diagnosis of breast cancer according to an embodiment of the present invention;
- FIG. 2: is an exploded perspective view illustrating the entire construction of the sensor assembly for self-diagnosis of breast cancer;
- FIG. 3: is a perspective view illustrating the assembled state of the sensor assembly for self-diagnosis of breast cancer;
- FIG. 4: is a side view of the sensor assembly for self-diagnosis of breast cancer; and
- FIGS. 5A and 5B: are plan and bottom views of the sensor assembly for self-diagnosis of breast cancer.

### DETAILED DESCRIPTION OF THE INVENTION

In the following preferred embodiments of the present invention will be described in detail with reference to the accompanying drawings to allow those skilled in the art to easily implement the present invention. In the following description, however, it is to be noted that the description of functions or configurations of conventional elements will be omitted to prevent making the gist of the present invention unclear. The same reference numerals refer to similar elements throughout the drawings.

It will be understood that when an element is referred to as being "connected to" another element, it can be "directly connected to" the other element or it can be "indirectly connected to" the other element with a further element interposed there between. Further, it will be further understood that the terms "includes" and/or "including" when used in this specification, specify the presence of stated features, but do not preclude the presence or addition of one or more other features.

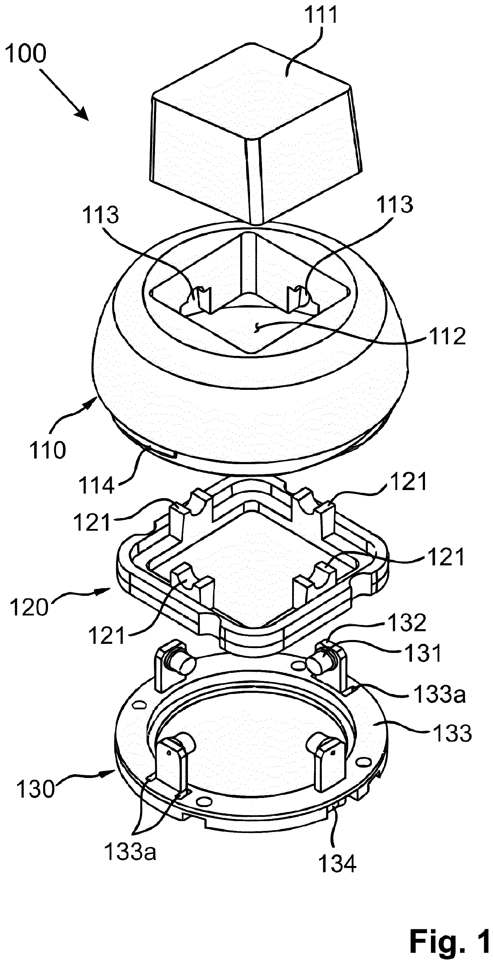

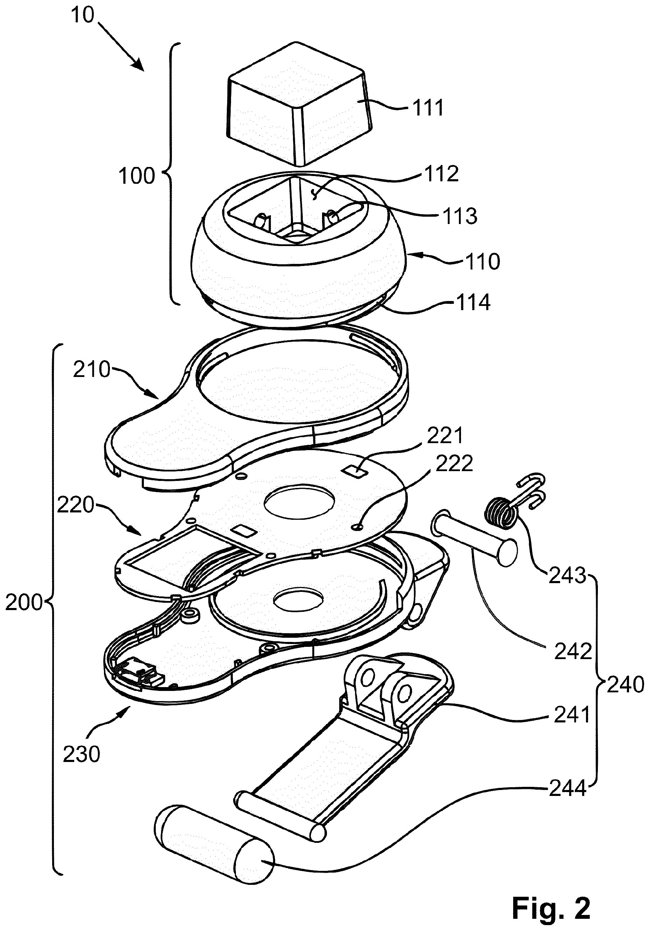

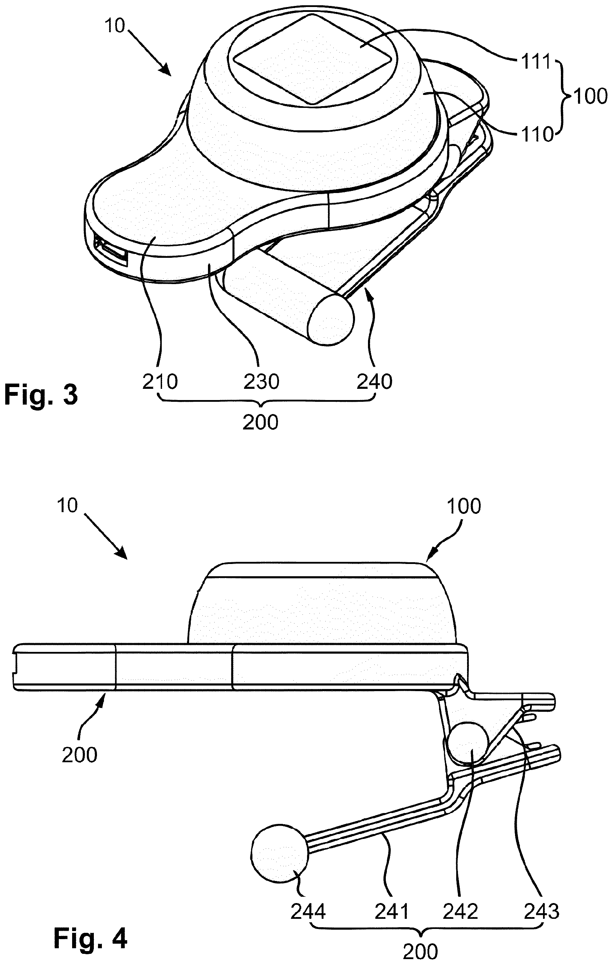

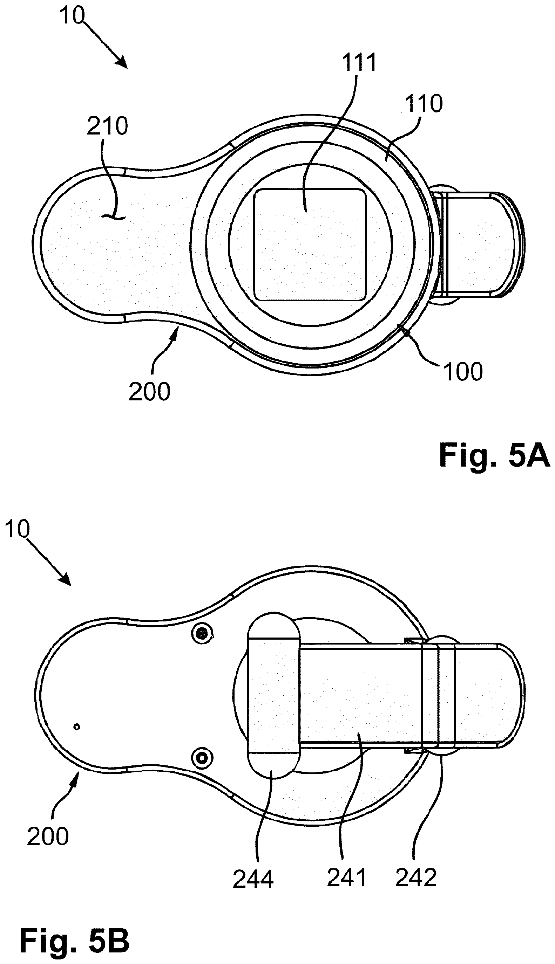

FIG. 1 is an exploded perspective view illustrating a poly-di-methyl-siloxane (PDMS) module of a sensor assembly for self-diagnosis of breast cancer according to an embodiment of the present invention. FIG. 2 is an exploded perspective view illustrating the entire construction of the sensor assembly for self-diagnosis of breast cancer. FIG. 3 is a perspective view illustrating the assembled state of the sensor assembly for self-diagnosis of breast cancer. FIG. 4 is a side view of the sensor assembly for self-diagnosis of breast cancer. FIGS. 5A and 5B are plan and bottom views of the sensor assembly for self-diagnosis of breast cancer. As illustrated in FIGS. 1 to 5, the sensor assembly 10 for self-diagnosis of breast cancer may include the PDMS module 100 and the mounting module 200.

The PDMS module 100 is a sensor module which senses elasticity distribution of breast tissue and emits near-infrared rays to allow a mobile analyzing device to obtain and process tactile images and near-infrared images for self-diagnosis of breast cancer. As illustrated in FIG. 1, the PDMS module 100 module may include a streamlined casing 110, an LED holder frame 120, and an LED-mounting frame 130.

The casing 110 has a tactile sensor 111 mounted in a rectangular recess 112 of the casing, a plurality of first LED holders 113 respectively provided in four inner walls of the rectangular recess 112, and a screw part 114 provided on a lower side of the casing 110 so as to be screw-coupled with the mounting module 200 to be described later.

The LED holder frame 120 is fixedly inserted into the casing 110 and has a plurality of second LED holders 121 corresponding to the first LED holders 113.

The LED-mounting frame 130 is fixedly inserted into the casing 110 to which the LED holder frame 120 has been mounted, and has four LED brackets 132 to which four near-infrared LEDs 131 are respectively mounted to emit near-infrared rays towards breast tissue so as to obtain near-infrared images of the breast tissue, a power connecting PCB plate 133 to which the four LED brackets are mounted, and a fixing protrusion 134 provided on a lower side of the power connecting PCB plate 133.

The first LED holders 113 and the second LED holders 121 serve to maintain a direction of near-infrared rays emitted from the near-infrared LEDs 131 without scattering and leaking of the near-infrared rays. That is, the first LED holders 113 and the second LED holders serve as passages to allow the near-infrared rays to be guided towards the tactile sensor 111.

The first LED holder 113 and the second LED holder 121 vertically join together to form an LED-mounting hole through which the near-infrared LED 131 mounted to the LED bracket 132 is fixedly held without additional components. The four near-infrared LEDs 131 are fixedly mounted at 90 degree angles to the LED brackets 132, so that the four near-infrared LEDs can emit near-infrared rays towards the tactile sensor 111 of the PDMS module 100 at the same height and direction.

The power connecting PCB plate 133 of the LED-mounting frame 130 is additionally provided with contact electrodes 133a, which are to be connected to a power source of the mounting module when the PDMS module is screw-coupled with the mounting module through the screw part 114 of the casing 110. Here, the contact electrodes may be disconnected from the power source of the mounting module when the PDMS module is unscrewed from the mounting module. Although the sensor assembly of the present invention has a separate power ON/OFF switch (not shown), when the PDMS module 100 is unscrewed from the mounting module, the power is turned off so as to save the battery power.

The fixing protrusion 134 of the LED-mounting frame 130 serves to allow the connection between contact electrodes between the PDMS module and the mounting module at the coupling-stop position when the PDMS module is screw-coupled to the mounting module.

The mounting module 200 is a module for attaching a mobile analyzing device. The mounting module 200 can be detachably coupled with the PDMS module 100 in a screw-coupling manner in which the mounting module can wirelessly supply an operating power to the PDMS module through contact between electrodes. As illustrated in FIG. 2, the mounting module 200 includes an upper casing part 210, a base frame 230 coupled to the upper casing part 210, and a main PCB plate 220 accommodated between the upper casing part 210 and the base frame. The upper casing part 210 has a streamlined shape, and is detachable screw-coupled with the screw part 114 of the PDMS module 100. The main PCB plate 220 has a shape corresponding to the upper casing part 210. The main PCB plate 220 has electrodes 221 for supplying an operating power to the PDMS module 100, and fixing holes 222 into which the fixing protrusions 134 are positively inserted. The base frame 230 accommodates the main PCB plate 220 when coupled to the upper casing part 210.

The mounting module 200 additionally includes a clip-type device holder 240 fastened to one side of the base frame 230 to hold a mobile analyzing device. The device holder 240 may have a holding plate, a connection part 141 provided on one side of the holding plate so as to be fastened to one side of the base frame 230, a connection pin 242 connecting the connection part 241 and the connecting side of the base frame 230, a spring member 243 coupled around the connection pin 242 to provide a coupling force to resiliently grip the mobile analyzing device, and a support part 244 provided on the other side of the holding plate to support the mobile analyzing device. Here, the mobile analyzing device to be held by the device holder 240 is a mobile terminal which has a camera unit for picking up tactile images and bear-infrared images, and a program engine (application) for processing combined images of the tactile images and the near-infrared images to self-diagnose breast cancer.

FIG. 3 is a perspective view illustrating the assembled state of the sensor assembly for self-diagnosis of breast cancer, and FIG. 4 is a side view of the sensor assembly for self-diagnosis of breast cancer. FIGS. 5A and 5B are plan and bottom views of the sensor assembly for self-diagnosis of breast cancer, wherein FIG. 5A shows the sensor assembly in a plan view, and FIG. 5B shows the sensor assembly in a bottom view.

According to the present invention, the sensor assembly for self-diagnosis of breast cancer is configured such that the sensor assembly is attached to a skin around user's breast to self-diagnose breast cancer, and the sensor assembly includes the PDMS module and the mounting module to which the PDMS module is detachably mounted in a screw-coupling manner in which the mounting module wirelessly supplies an operating power to the PDMS module through contact between electrodes, thereby providing simple structure and ease use of the sensor assembly.

Further, according to the present invention, the sensor assembly includes the PDMS module and the mounting module to which the PDMS module is detachably mounted in a screw-coupling manner in which the mounting module wirelessly supplies an operating power to the PDMS module through contact between electrodes, thereby having the effects that the sensor assembly can be easily used, and when in not use, simple screw-detachment of the PDMS module from the mounting module enables the PDMS module to be turned off to save power.

Although a preferred embodiment of the present invention has been described for illustrative purposes, those skilled in the art will appreciate that various modifications, additions and substitutions are possible, without departing from the scope and spirit of the invention as disclosed in the accompanying claims.

### Table of Reference Numbers:

- 10: Sensor assembly
- 100: PDMS Module
- 110: Casing
- 111: Tactile sensor
- 112: Rectangular recess
- 113: First LED holder
- 114: Screw part
- 120: LED holder frame
- 130: LED-mounting frame
- 131: Near-infrared LEDs
- 132: LED brackets
- 133: PCB plate
- 133a: Electrode
- 134: Protrusion
- 141: Connection part

- 200: Mounting module
- 210: Upper casing part
- 220: PCB plate
- 221: Electrode
- 222: Fixing hole
- 230: Base frame
- 240: Clip-type device holder
- 241: Connection part
- 242: Connection pin
- 243: Spring member
- 244: Support plate

## Claims

1. A sensor assembly (10) for self-diagnosis of breast cancer, the sensor assembly comprising:
• a poly-di-methyl-siloxane (PDMS) module (100) sensing elasticity distribution of breast tissue and emitting near-infrared rays to allow a mobile analyzing device to obtain and process tactile images and near-infrared images for self-diagnosis of breast cancer; and
• a mounting module (200) to which the PDMS module (100) is detachably mounted in a screw-coupling manner in which the mounting module wirelessly supplies an operating power to the PDMS module through contact between electrodes.

2. The Sensor assembly (10) of claim 1, wherein the PDMS module (100) includes:
• a streamlined casing (110) having a tactile sensor (111) mounted in a rectangular recess (112) of the casing,
• a plurality of first LED holders (113) respectively provided in four inner walls of the rectangular recess (112), and
• a screw part (114) provided on a lower side of the casing so as to be screw-coupled with the mounting module (200);
• an LED holder frame (120) fixedly inserted into the casing (110) and having a plurality of second LED holders (121) corresponding to the first LED holders (113); and
• an LED-mounting frame (130) fixedly inserted into the casing (110) to which the LED holder frame (120) has been mounted, and having four LED brackets (132) to which four near-infrared LEDs (131) are respectively mounted to emit near-infrared rays towards breast tissue so as to obtain near-infrared images of the breast tissue,
• a power connecting PCB plate (133) to which the four LED brackets (132) are mounted, and a fixing protrusion (134) provided on a lower side of the power connecting PCB plate (133).

3. The sensor assembly of claim 2, wherein the first LED holders (113) and the second LED holders (121) serve to maintain a direction of near-infrared rays emitted from the near-infrared LEDs (131) without scattering and leaking of the near-infrared rays.

4. The sensor assembly of claim 2 or claim 3, wherein the first LED holder (113) and the second LED holder (121) vertically join together to form an LED-mounting hole through which the near-infrared LED (131) mounted to the LED bracket (132) is fixedly held without additional components.

5. The sensor assembly of one of the claims 2 to 4, wherein the four near-infrared LEDs (131) are fixedly mounted at 90 degree angles to the LED brackets (132), so that the four near-infrared LEDs are able to emit near-infrared rays towards the tactile sensor (111) of the PDMS module (100) at the same height and direction.

6. The sensor assembly of one of the claims 2 to 5, wherein the power connecting PCB plate (133) of the LED-mounting frame (130) is additionally provided with contact electrodes (133a), which are to be connected to a power source of the mounting module (200) when the PDMS module is screw-coupled with the mounting module (200) through the screw part (114) of the casing (110), and the contact electrodes are to be disconnected from the power source of the mounting module when the PDMS module (100) is unscrewed from the mounting module.

7. The sensor assembly of one of claims 2 to 6, wherein the mounting module (200) includes:
• an upper casing part (210) having a streamlined shape, the upper casing part being detachably screw-coupled with the screw part (114) of the PDMS module (100);
• a main PCB plate (220) disposed in the upper casing part (210) while having a shape corresponding to the upper casing part (210), the main PCB plate (220) having an electrode (221) for supplying an operating power to the PDMS module (100), and a fixing hole (222) into which the fixing protrusion (134) is positively inserted; and
• a base frame (230) accommodating the main PCB plate (220) when coupled to the upper casing part (210).

8. The sensor assembly of claim 7, wherein the mounting module (200) additionally includes a clip-type device holder (240) fastened to one side of the base frame (230) to hold a mobile analyzing device.
